# EUROPEAN PATENT APPLICATION

(11) **EP 1 618 876 A1**
(43) Date of publication of application: **25.01.2006**
(21) Application number: 04077091.9
(22) Date of filing: 19.07.2004
(51) Int. Cl.: A61K 31/133, A61K 31/688, A23J 7/00, A23L 1/30, A61P 9/10

(54) **Use of sphingolipids for prevention and treatment of atherosclerosis**

(71) Applicant: Nederlandse Organisatie voor Toegepast-Natuurwetenschappelijk Onderzoek TNO, 2628 VK Delft (NL)
(72) Inventor: Nieuwenhuizen, Willem Ferdinand, 3981 AM Bunnik (NL); Emeis, Josephus Jan, 1018 BE Amsterdam (NL); Havekes, Aloysius Maria, 2402 PP Alphen aan den Rijn (NL)
(74) Representative: Winckels, Johannes Hubertus F.

(57) **Abstract**

The present invention is in the field of prevention and treatment of atherosclerosis and atherosclerosis-related clinical conditions. In particular, the present invention relates to the use of sphingolipids, more preferably phytosphingosine, sphingosine, sphinganine, ceramide, cerebroside and/or sphingomyelin for prevention and treatment of atherosclerosis in a subject and to the use of sphingolipids as an anti-atherogenic agent. The invention also encompasses methods of treatment of subjects suffering from atherosclerosis.

## Description

### FIELD OF THE INVENTION

The present invention is in the field of prevention and treatment of atherosclerosis. In particular, the present invention relates to the use of specific sphingolipids, more preferably phytosphingosine, sphingosine and/or sphinganine for prevention and/or treatment of atherosclerosis and its clinical symptoms, and to the use of sphingolipids for the manufacture of a medicament, food item or food supplement for the prevention and/or treatment of atherosclerosis.

### BACKGROUND OF THE INVENTION

Heart attack and stroke are among the primary causes of death in Western societies. These cardiovascular diseases (CVD) are principally caused by atherosclerosis, a process in which so called plaques at the luminal side of arteries are formed. Depending on how advanced these plaques are, the plaques consist amongst others, of lipid loaded cells of the immune system (such as T-cells and macrophages), smooth muscle cells, connective tissue, cholesterol crystals, and calcium. Plaques may hamper the blood flow through the arteries since they narrow the lumen of the latter. Plaques are often covered with a cap of endothelial cells and protein, covering the proagulant core of the plaque. In a so-called unstable plaque the cap may rupture, thus exposing the proagulant core to the coagulation system of the blood. This may lead to an instant blockage of the artery lumen, causing clinical conditions such as an acute myocardial infarction, or stroke. Destabilisation of plaques may be caused by activation of T-cells and macrophages in the plaque area such as is the case in inflammatory conditions.

Atherosclerosis is a complex process, the precise aetiology of which remains unknown. Compelling evidence now points to vascular inflammation and resultant oxidative stress as important triggers in the complex chain of events leading to atherosclerosis. Oxidative damage to the endothelial layer of the artery causes fats, cholesterol, platelets, fibrin, cellular debris and calcium to be deposited in the arterial wall. These substances appear to stimulate the endothelial cells to produce other materials that result in further accumulation of cells in the vascular intima, forming a lesion. These cells accumulate and many of them divide. At the same time, lipid accumulates within and around the cells.

A crucial initiating event in atherosclerosis is the interaction of white blood cells―monocytes and lymphocytes―with the endothelium, which is the cell layer of the blood vessel wall that faces the blood stream. Following adherence of these white blood cells to the endothelium, they migrate across the endothelium and into the arterial wall. Once trapped in the arterial wall, the monocytes engorge oxidized ("bad") lipoproteins and are converted into fat-laden foam cells. Formation and aggregation of foam cells is the first manifestation of atherosclerosis, leading to the narrowing of the arterial lumen and, eventually, to advanced CVD.

The adherence of monocytes to the endothelium and subsequent migration into the arterial wall are caused by presentation of "sticky" proteins called cellular adhesion molecules on the surface of endothelial cells. Inflammatory cytokines directly stimulate the production of these adhesion molecules.

A strong indication of inflammation in the aetiology of atherosclerosis is the presence of C-reactive protein (CRP) in the blood of patients with heart disease. Plaque formation is also associated with increased levels in the aorta of a protein called NF-κB that controls the formation of cytokines and stimulates the growth of cells.

It is known that several cell types take part in plaque inflammation, including dendritic cells, macrophages, mast cells and T-lymphocytes. The role of T-cells in atherosclerosis has been well reviewed (de Boer et al., Cardiovascular Research 60 (2003) 78-86). The participation of the T-lymphocytes or T-cells in atherogenesis is relevant for several reasons. Activated T-cells expand clonally and actively secrete cytokines or express specific receptors. These receptors and cytokines modulate the type and intensity of the inflammatory response, and affect the growth of vascular smooth muscle cells and the synthesis and degradation of extracellular matrix proteins. Thus, the activation of T cells may affect the stability of the plaque. The clonal proliferation and cytokine production, in turn activates other plaque-associated cells, particularly macrophages.

Immunohistochemical studies have revealed clusters of inflammatory cells, including activated T-cells, in the proximity of newly formed capillary blood vessels in advanced plaques lined with activated endothelium, suggesting that at these sites indeed a local inflammatory burst is initiated. The importance of the process of T-cell activation in advanced atherosclerotic plaques stems mainly from clinico―pathologic observations, on lesions derived from patients with symptomatic coronary artery disease. Active inflammation, in which T-cells participate, is a common finding at rupture sites of thrombosed coronary plaques of patients who died of acute myocardial infarction. Further, in the plaque tissue of patients with stable coronary artery disease only a small percentage of the T-cells appeared to be activated. On the other hand, in the culprit lesions from patients with unstable coronary syndromes, and particularly those with myocardial infarction, the frequency of activated T-cells, assessed by expression of several activation markers was increased significantly. Such findings suggest that around the time of onset of acute syndromes (which may be days to weeks) substantial T-cell activation takes place, with as a consequence, tissue-remodeling activity.

On the basis of the above, it can be concluded that e.g. T-cells and macrophages, activated by inflammation, play a role both in the aetiology of atherosclerosis and in the (in)stability of plaques, which is important for the clinical consequences of atherosclerosis.

Thus, the deactivation of activated cells, and particularly of T-cells involved in atherosclerosis, would provide for a to treatment modality that interferes with the process of plaque inflammation. It follows that such reduction of inflammation within the arteries may inhibit plaque build-up and plaque destabilisation and thereby reduce the risk of atherosclerosis and associated clinical conditions.

### SUMMARY OF THE INVENTION

The present inventors have now surprisingly found that sphingolipids exhibit an anti-inflammatory effect and may thus be used in anti-atherogenic therapies and the prevention of the clinical complications of atherosclerosis.

In a co-pending application (PCT/NL2004/00048), the present inventors have previously described that sphingolipids have a cholesterol and triglyceride-lowering effect. The presently described anti-inflammatory effect is exhibited separately from the lipid lowering effect of the sphingolipid compounds. Phytosterols used in food to lower cholesterol (and not triglycerides), do not exhibit the observed anti-inflammatory effect.

For example, release of TNFα in LPS-stimulated macrophages is strongly reduced when these macrophages are brought into contact with sphingolipids, specifically with phytosphingosine, sphingosine and/or sphinganine. Also, and perhaps more importantly, the (phorbol 12- myristate 13-acetate) PMA/ionomycin-induced activation of the (Nuclear Factor of Activated T-cells) NFAT-mediated signal transduction pathway in T-cells is strongly suppressed after exposure of these cells to a concentration of 1 µg/mL of sphingolipid compared to control cells, while at 10 µg/mL this suppression is even complete. As sphingolipids are natural compounds found in all eukaryotic cells, this paves the way for the preparation of foodstuffs and clinically safe medicaments without undesirable side effects, with a capacity to prevent and/or treat atherosclerosis and its sequellae in mammals, specifically in humans.

When in the context of the present invention reference is made to "anti-atherogenic effect" of a compound, this should be understood as the effect by which the inflammatory responses of cells or processes associated with atherosclerosis are prevented and/or reduced, preferably in T-cells. Such an anti-inflammatory effect is thus anti-atherogenic and prevents clinical conditions which are caused by atherosclerosis; and is displayed separate from other effects of that compound that may also contribute to lowering the risk or atherosclerosis such as the lipid lowering effect of sphingolipids. Also "preventing and/or treating atherosclerosis" should be understood as preventing and/or treating atherosclerosis by lowering the inflammatory reaction of the body that is associated with the pathogenesis and pathophysiology of atherosclerosis.

In one aspect the invention now provides the use of a sphingolipid according to the formula (I) 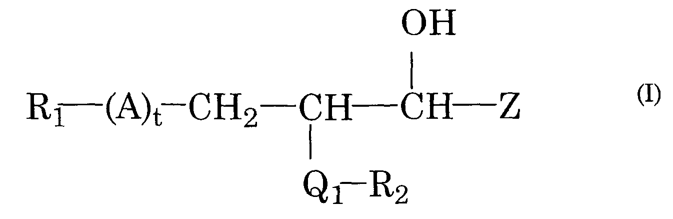
wherein
Z is R₃ or -CH(OH)-R₃;
A is sulphate, sulphonate, phosphate, phosphonate or -C(O)O-;
R₁ is H, hydroxyl, alditol, aldose, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-); preferably an secondary amine group; and t is 0 or 1, or a precursor, a derivative or a pharmaceutically acceptable salt thereof for the manufacture of a medicament for preventing and/or treating atherosclerosis.

In a preferred embodiment, said sphingolipid is a sphingolipid according to the formula (II) 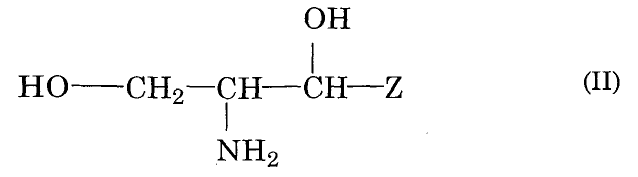
wherein
Z is R₃ or CH(OH)-R₃ and R₃ is an unsaturated or saturated (C₁-C₃₀) alkyl chain, even more preferably a sphingolipid according to formula (III) 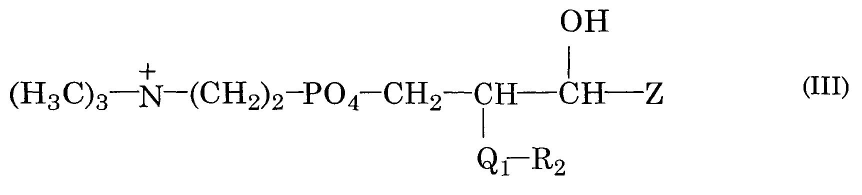
wherein
Z is R₃ or CH(OH)-R₃, preferably R₃, and R₃ is an unsaturated or saturated (C₁-C₃₀) alkyl chain, preferably R₃ is an unsaturated (C₁-C₃₀) alkyl chain;
Q₁ is a primary amine group (-NH₂), a secondary amine group (-NH-) or an amide group (-NH-CO-); preferably an amine group, and
R₂ is H or unsaturated or saturated (C₁-C₃₀) alkyl chain.

In highly preferred embodiments, wherein the sphingolipid is a sphingolipid according to the formula (II), a sphingolipid according to the present invention is phytosphingosine, sphinganine or sphingosine, and in another highly preferred embodiment, wherein the sphingolipid is a sphingolipid according to the formula (III), said sphingolipid is sphingomyelin.

The present invention also provides use of a sphingolipid according to the formula (I), (II) or (III) or a precursor or a derivative in food items as an anti-atherogenic agent and/or as an agent preventing the clinical complications of atherosclerosis.

In another aspect, the present invention provides a method for preventing atherosclerosis in a healthy subject, comprising providing said subject a diet with enhanced levels of a sphingolipid according to the formula (I), (II) or (III) or a precursor, a derivative or a pharmaceutically acceptable salt thereof.

In yet another aspect, the present invention provides a method of treatment of subjects suffering from atherosclerosis, said method comprising administering to subjects in need thereof a therapeutically effective amount of a pharmaceutical composition, said composition comprising a sphingolipid according to the formula (I), (II) or (III), or a precursor, a derivative or a pharmaceutically acceptable salt thereof and a pharmaceutically acceptable carrier, and optionally one or more excipients.

In yet another aspect, the present invention relates to the use of a food product comprising enhanced levels of a sphingolipid according to the formula (I), (II) or (III) or a precursor, a derivative or a pharmaceutically acceptable salt thereof, in a diet for preventing or treating atherosclerosis and its clinical complications such as plaque rupture.

### DESCRIPTION OF THE DRAWINGS

Figure 1 shows the reduced release of TNFα by LPS-stimulated macrophages as a result of sphingolipids.
Figure 2 shows the influence of sphingosine, sphinganine and phytosphingosine on the NFAT-mediated luciferase activity (i.e. T-cell activation).

### DETAILED DESCRIPTION OF THE INVENTION

With the term "inflammatory process associated with atherosclerosis" is meant the inflammatory reaction of the body that is associated with an increased risk of acquiring atherosclerosis, with the pathogenesis of atherosclerosis (i.e. the generation and development of the disease) and/or with the pathophysiology of atherosclerosis (i.e. the physiological manifestations associated with or resulting from the disease).

As used herein, the term "sphingolipid" includes the generally accepted term of this particular lipid-like compound, but it is specifically used to address the group of compounds according to the formulas (I), (II) and (III) of the present invention, including analogs or derivatives or pharmaceutically acceptable salts thereof, alone, or in combination, or as a so-called precursor compound, unless explicitly noted otherwise.

The term "therapeutically effective amount" as used herein refers to an amount of a therapeutic agent to treat, ameliorate, or prevent a desired disease or condition, or to exhibit a detectable therapeutic or prophylactic effect. The precise effective amount needed for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation.

A "derivative", "analog" or "analogue" is defined herein as a sphingolipid according to the formula (I), (II) or (III) that is subjected to a (bio)chemical modification (e.g. organo-chemical or enzymatical). Derivatising may comprise the substitution of certain chemical groups to the sphingolipid, thereby retaining the sphingolipid character of the compound. Such derivatizations are known in the art. The derivatives and analogues maintain the biological activity of the natural sphingolipid and act in a comparable way, but may provide advantages to the molecule such as longer half-life, resistance to degradation or an increased activity. A very suitable derivative for phytosphingosine is for instance TAPS (see below). Such a derivative may suitably be used in embodiments of the present invention since after hydrolysis, for instance in the body, the converted compound will exert its anti-atherogenic effect.

A "pharmaceutically acceptable salt" is defined herein as a salt wherein the desired biological activity of the sphingolipid is maintained and which exhibits a minimum of undesired toxicological effects. Non-limiting examples of such a salt are (a) acid addition salts formed with inorganic acids (e.g., hydrochloric acid, hydrobromic acid, sulphuric acid, phosphoric acid, nitric acid, and the like), and salts formed with organic acids (such as e.g. acetic acid, oxalic acid, tartaric acid, succinic acid, malic acid, ascorbic acid, benzoic acid, tannic acid, palmitic acid, polyglutamic acid, naphthalene sulphonic acid, naphthalene disulphonic acid, polygalacturonic acid and the like); (b) base addition salts formed with metal cations such as zinc, calcium, bismuth, barium, magnesium, aluminium, copper, cobalt, nickel, cadmium, sodium, potassium and the like, or with a cation formed from ammonia, N,N-dibenzylethylenediamine, D-glucosamine, tetraethylammonium or ethylenediamine; or (c) combinations of (a) and (b); e.g. a zinc tannate or the like. The use of a pharmaceutically acceptable salt of a sphingolipid according to the formula (I), (II) or (III), such as an ammonium salt or a chloride salt is preferred since the salt form is better soluble and will thus enhance the bio-availability of the sphingolipid. Preferably a salt of HCl is used. The use of a pharmaceutically acceptable salt is not limited to pharmaceutical preparations, but includes the use in food items or food supplements.

A "precursor" is defined herein as a derivative of the active compound with similar, less or no activity, and which can be transformed to the active compound e.g. by the digestive tract or other digestive systems in the body. Such precursors can be obtained by chemical or enzymatic modification of the active molecule.

"Subject" as used herein includes, but is not limited to, mammals, including, e.g., a human, non-human primate, mouse, pig, cow, goat, cat, rabbit, rat, guinea pig, hamster, horse, monkey, sheep, or other non-human mammal; and non-mammal animals, including, e.g., a non-mammalian vertebrate, such as a bird (e.g., a chicken or duck) or a fish, and an invertebrate.

Sphingolipids are lipids of which some occur in food in low concentrations and which form a minor but important constituent of the cells of plants, animals and man. Since several sphingolipids occur naturally in the body of man and animal, they will be easily acceptable for addition to food compounds or as pharmaceutical agents.

Sphingolipids are generally composed of a long sphingoid base (sphingosine, sphinganine, phytosphingosine, or a related compound) as the central group of the molecule or "backbone" (see *intra. alia.* Karlsson. 1970. Chem. Phys. Lipids, 5:6-43), which comprises an amide-linked long-chain fatty acid and a head group. There are hundreds of known classes of sphingolipids with different head groups (e.g. cholinephosphate, glucose, galactose, polysaccharides) and with different fatty acids and sphingoid bases (see *intra alia* Merrill & Sweeley. 1996. New Comprehensive Biochemistry: Biochemistry of Lipids, Lipoproteins, and Membranes, (Vance, D.E. & Vance, J.E., eds.), pp. 309-338, Elsevier Science, Amsterdam).

The simplest sphingolipids, like sphingosine and sphinganine normally occur in food in very low concentrations. The richest sources of sphingolipids are dairy products, soy beans, eggs, meat, including fish meat, shellfish meat and meat of marine invertebrates, such as starfish. The most abundant sphingolipids in our food are sphingomyelin (milk and eggs) and ceramide (meat). Whole milk contains predominantly sphingomyelin, but also contains glucosylceramide, lactosylceramide and gangliosides. Potato, apple, tomato, spinach, pepper and rice especially contain cerebrosides in low concentration (see, e.g. Stryer L., Biochemistry, [W.H. Freeman and Co., NY, USA[, 1988, p. 287 and Ryu J, Kim JS, Kang SS., Cerebrosides from Longan Arillus. Arch Pharm Res. 2003 Feb;26(2):138-42; Kawatake S, Nakamura K, Inagaki M, Higuchi R. Isolation and structure determination of six glucocerebrosides from the starfish *Luidia maculata.* Chem Pharm Bull (Tokyo) 2002 Aug;50(8):1091-6).

It is known that sphingosine and sphingosine-analogs inhibit growth and metastasis of human and animal tumor cells (see e.g. EP 0 381 514). It is also known that administration of sphingomyelin to the food of rats can significantly decrease the chances of occurrence of malignant, chemically induced colon cancer (see Schmeltz, E., *et al*.,).

Sphingolipids are also used in pharmaceutical compositions to protect skin and/or hair against the damaging effects of air pollution (see e.g. US 5.869.034).

The antimicrobial action of sphingosine as a component of the skin against bacteria such as *Staphylococcus aureus, Candida albicans* and *Propionibacterium acnes* is known from dermatology (Bibel et al. 1992. J. Invest. Dermatol. 98(3):269-73; Bibel *et al.* 1995. Clin Exp Dermatol 20(5):395-400), and the application of topical ointments comprising sphingosine is described therein.

The present inventors have now found a T-cell deactivating effect of sphingolipids, which effect will result in a prevention or reduction of the inflammatory response associated with T-cell activation. As such the effect observed is an anti-inflammatory effect and the invention thus relates to the use of the sphingolipids in order to exert this effect in a person in need thereof. Therefore, the effect found by the present inventors relates to prevention or reduction of the inflammatory responses, particularly those associated with T-cell activation, such as present in the aetiology of atherosclerosis and its clinical manifestations. Therefore the present invention relates to anti-atherogenic and/or anti-atherosclerotic properties of sphingolipids.

The invention now relates to the use of sphingolipids for the prevention and/or treatment of atherosclerosis; and to the prevention of atherosclerosis-related clinical conditions in a subject. The sphingolipid may be administered to said subject in the form of a food item, food supplement or medicament.

The present invention in a first aspect relates to the use of a sphingolipid or a precursor, a derivative or a pharmaceutically acceptable salt thereof with the general formula: 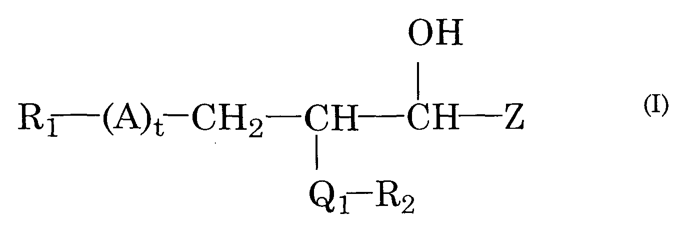
wherein
Z is R₃ or -CH(OH)-R₃;
A is sulphate, sulphonate, phosphate, phosphonate or -C(O)O-;
R₁ is H, hydroxyl, alditol, aldose, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), a secondary amine group (-NH-) or an amide group (-NH-CO-); and
t is 0 or 1,
for the manufacture of a medicament for preventing and/or treating atherosclerosis.

R₁ can be selected from aldose radicals such as radicals of acesulfam, allose, altrose, arabinose, erythrose, fructose, fucose, galactose, glucose, gulose, idose, isomaltose, lactose, lyxose, maltose, mannose, melezitose, psicose, raffinose, rhamnose, ribose, saccharose, sorbose, stachyose, sucrose, tagatose, talose, threose, trehalose, turanose, xylose and xylulose, and other mono-, di-, or polysaccharides.

R₁ is preferably selected from amino acids radicals, such as radicals of alanine, arginine, asparagines, aspartate, carnitine, citrulline, cysteine, cystine, GABA, glutamate, glutamine, gluthathione, glycine, histidine, hydroxyproline, isoleucine, leucine, lysine, methionine, ornithine, phenylalanine, proline, serine, taurine, threonine, tryptophane, tyrosine and valine or derivatives or combinations thereof.

R₁ is more preferably selected from the group consisting of hydrogen, hydroxyl or hydroxyl-containing group (e.g. hydroxyalkyl), alditol radical or polyol radical, such as radicals of adonitol, arabitol, dulcitol, erythritol, ethyleneglycol, glycerol, inositol, lactitol, maltitol, mannitol, propyleneglycol, ribitol, sorbitol, threitol and xylitol, and of methanol, ethanol, ethanediol, isopropanol, n-propanol, 1,3-propanediol, and other poly-alcohols.

Even more preferably R₁ is selected from the group consisting of radicals of alcohols such as, choline, ethanolamine, ethanol, glycerol, inositol, tyrosine and serine and still more preferably from the alcohol moieties of phosphoglycerides or phosphoglyceride-alcohols, such as choline, serine, ethanolamine, glycerol or inositol.

R₁ is most preferably a hydroxyl group.
(A) can have any desired counter-ion for the formation of a salt of a sphingolipid according to the formula (I).

It is possible that the amino group such as may be present in the form of Q₁ in a sphingolipid according to the formula (I) is modified, e.g. by single or multiple methylation, alkylation, acylation of acetylation or by modification to a formic acid amide.

Also the free hydroxyl groups in the formula (I), specifically those in R₃ may be modified in ways known to the skilled person.

Further, all possible racemates and (dia)stereoisomers of a sphingolipid according to the formula (I) can be used in the present invention. It is possible to use compounds according to the formula (I) wherein Q₁ is substituted by e.g. H, a hydroxyl, a carboxyl or a cyano group. Preferred is a compound wherein Q₁ is the amino group.

R₂ is H or unsaturated or saturated (C₁-C₃₀) alkyl chain and R₃ is unsaturated or saturated (C₁-C₃₀) alkyl chain.

The term alkyl as used herein refers to a saturated or unsaturated straight chain, branched or cyclic, primary, secondary or tertiary hydrocarbon of C₁- C₃₀, optionally substituted, and comprises specifically methyl, ethyl, propyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl and 2,3-dimethylbutyl, heptyl, octyl, nonyl, decyl, undecyl, dodecyl, tridecyl, tetradecyl, pentadecyl, hexadecyl, heptadecyl, octadecyl, nonadecyl, eikosyl, heneikosyl and dokosyl and isomers thereof.

The C₁- C₃₀ alkyl chain or -group may be optionally substituted with one or more groups selected from the collection consisting of hydroxyl, amino, alkylamino, arylamino, alkoxy, aryloxy, nitro, cyano, sulphonic acid, sulphate, sulphonate, phosphonate or phosphate, either unprotected or protected insofar as desired. These substitutes are known to the person skilled in the art, for example from Greene et al., Protective Groups in Organic Synthesis, John Wiley & Sons, 2^{nd} Edition, 1991. Preferred embodiments of C₁- C₃₀ alkyl chains constitute C₈-C₂₄ alkyl chains.

A compound of the formula (I) is a sphingolipid, or a precursor, a derivative or pharmaceutically acceptable salt thereof.

Even more preferably, in a compound according to the formula (I), or a precursor, a derivative or a pharmaceutically acceptable salt thereof, R₁ is a hydroxyl group, t is 0, R₂ is hydrogen, R₃ is unsaturated or saturated (C₁-C₃₀) alkyl, Q₁-R₂ together is an amine group. More preferably therefore, a sphingolipid used in embodiments of the present invention is a sphingolipid with the general formula (II): 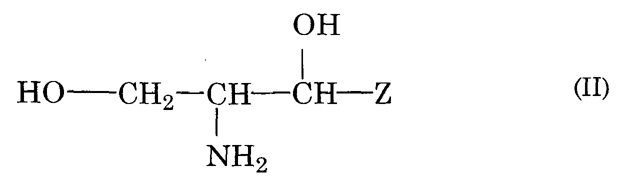
wherein and Z is R₃ or CH(OH)-R₃ and R₃ is an unsaturated or saturated (C₁-C₃₀) alkyl chain.

In a most preferred embodiments of the present invention, a phytosphingosine, sphingosine and/or sphinganine is used, since these compounds show excellent anti-inflammatory activity and are therefore very suitable for the prevention and treatment of atherosclerosis, and thus prevention of its clinical sequellae claimed in the present invention.

Besides phytosphingosine, sphingosine and/or sphinganine, also derivatives of these compounds may be used in aspects of the present invention. For instance, in stead of a hydroxyl headgroup, a choline phosphate, ethanolamine phosphate, serine phosphate, inositol phosphate, glycerol phosphate, glucose or galactose head group may be used as R₁ group in a compound according to the formula (I). Basically all headgroups within the definition of R1 above may be used for derivatization of phytosphingosine, sphingosine and sphinganine. A derivative such as lyso-sphingomyelin may also be used in embodiments of the present invention.

It is also possible to use a combination of sphingolipids according to the formula (I) and/or (II) and/or (III) in aspects of the present invention.

Other preferred sphingolipids for use in the present invention are ceramide, cerebroside and/or sphingomyelin, more preferably sphingomyelin and/or lyso-sphingomyelin, with any suitable sphingoid bases.

In principle, sphingolipids according to the formula (I) and/or (II) and/or (III) of all possible sources are suitable for use in aspects and embodiments of the present invention. For instance, a suitable sphingolipid such as phytosphingosine may be obtained from plants such as corn (Wright et al., Arch. Biochem. Biophys. 415(2), 184-192 and references therein), from animals (skin fibroblasts) or from microorganisms such as yeasts (such as *Pichia ciferrii).* The sphingolipids may be isolated from these organisms or can be used in a less pure form, i.e. as an enriched fraction, or in the case of microorganisms such as yeasts by taking the complete organism(s) or fractions thereof. Further, sphingolipids may be isolated from other suitable sources, such as from milk, egg, soy, yeast, bacteria, algae, plants, meat, brain, etc. or may be chemically or enzymatically prepared, for use in a pharmaceutical composition or food additive according to the invention.

For application in a food item or food supplement according to the present invention, a sphingolipid is preferably, but not necessarily, derived from a food-grade source. Examples of suitable food-grade sources are e.g. bakery yeast, brewers yeast and egg, and certain types of bacteria, (filamentous) fungi, sponges and algae, in particular, but not exclusively those species of bacteria, yeast and fungi which are generally recognized as safe (GRAS). Sphingolipids and related compounds from which suitable sphingolipids may be derived are produced by a large number of different genera of micro-organisms. Special reference in this context is made to US 6,204,006 wherein a large number of suitable bacterial sources of sphingolipids are described.

Sphingolipids may be derived from the above sources by methods known to the skilled person for instance by extraction with (organic) solvents, chromatographic separation, precipitation, crystallization and/or enzymatic of chemical hydrolysis. The production of a sphingolipid-enriched (specifically a sphingomyelin-enriched) fraction from milk is for instance known from WO94/18289. Sphingolipids may also be derived from fat concentrates of various animal products such as milk products, egg products and blood products such as known from US 5,677,472.

Methods for the preparation of sphingolipids and sphingolipid derivatives are *i.a.* known from EP 0 940 409, WO 98/03529, WO 99/50433 and US 6,204,006 and the artisan will be capable of preparing derivatives by these and other methods. Various routes for obtaining sphingosines are described by D. Shapiro in "Chemistry of Sphingolipids", Hermann, Paris (1969). Methods for producing certain phytosphingolipid derivatives are known to the skilled person, for instance it is known from US 6,204,006 and US 5,618,706 to derive tetraacetyl-phytosphingosine (TAPS) from microbial sources (i.e. *Pichia ciferrii)* and to subject this TAPS to hydrolysis to yield phytosphingosine.

A sphingolipid according to the formula (I), or a precursor, a derivative or a pharmaceutically acceptable salt thereof, may also be synthesized by known methods such as e.g. known from U.S. patents 5.232.837 and 5.110.987, or by standard modifications of these methods.

A known issue relating to the administration of sphingolipids, be it in foods or in pharmaceutical compositions, is that they can be metabolized. This is particularly relevant for application of sphingolipids in the digestive tract. This issue may be addressed by administering a sphingolipid according to the formula (I), more preferably according to formula (II) or (III), or a derivative or a pharmaceutically acceptable salt thereof, alone or in combination, as a so-called precursor compound which compound comprises certain substituents as a result of which the compound can no longer, or only at reduced rates, be metabolized. These precursors are preferably resistant to hydrolysis in the upper parts of the digestive tract (e.g. mouth, stomach), and are for instance split relatively easy in the lower part of the digestive tract (e.g. coecum, colon), if the sphingolipid should have its working especially there. It is also possible that activation occurs when the compound has been absorbed from the digestive tract, e.g. in the serum or the liver. As a result, the amount of the compound is raised at those locations where the sphingolipid has its action. For instance, a sphingolipid precursor may be used that can be split or activated *in vivo* by a suitable enzyme so that the sphingolipid is liberated and may exert it anti-atherogenic effect in the subject. Special reference in this context is made to WO 99/41266 wherein such sphingolipid precursors have been described.

It is possible to modify a precursor of a sphingolipid according to the formula (I), (II) or (III) by an *in situ* enzymatic or chemical conversion, i.e. in the body, to a sphingolipid according to the formula (I), (II) or (III), which can be used in embodiments of the present invention. Such precursors of a sphingolipid according to the formula (I), (II) or (III) are therefore also suited for use according to the invention. A condition is that the precursor is converted in the body, e.g. preferably in the intestine, to a sphingolipid according to the formula (I), (II) or (III), e.g. by enzymatic conversion, in which case there is *in situ* activation. It is therefore, for instance possible to administer together with e.g. sphingomyelin, the enzyme sphingomyelin deacylase which may convert the sphingomyelin to lyso-sphingomyelin. Another possibility is to use sphingomyelinase to convert sphingomyelin into ceramide. In its turn ceramide can be broken down by ceramidase into a sphingoid base structure and a fatty acid. Other examples of enzymes may for instance be found in Sueyoshi et al., 1997, J Lipid Res 38:1923-7. Preferably, however, the sphingolipid according to the formula (I), (II) or (III) is not used as a precursor but in its "active" form in a food item or a food supplement or a pharmaceutical preparation.

A sphingolipid according to the formula (I), (II) or (III), or a precursor, a derivative or a pharmaceutically acceptable salt thereof, may be provided to a subject in need thereof for prophylactic or therapeutic reasons. A sphingolipid according to the formula (I), (II) or (III), or a precursor, a derivative or a pharmaceutically acceptable salt thereof, may be provided to a subject in need thereof in the form of a food item or food supplement, or in the form of a pharmaceutical preparation, all such administration forms being capable of treating and/or preventing atherosclerosis in a subject. As a consequence of the prevention or treatment of atherosclerosis, also the clinical effects or sequellae of atherosclerosis will be prevented. Thus, the invention is also directed at the prevention of the clinical effects of atherosclerosis such as high blood pressure as a results of narrowing of the lumen of the arteries, effects from decreased blood flow through such arteries such as angina pectoris, acute myocardial infarctions, or cerebrosvascular accidents, and the rupture of atherosclerotic plaques which can cause thrombosis.

The present invention therefore also relates to a method of treating and/or preventing atherosclerosis and its clinical sequellae in a subject comprising providing said subject a diet with enhanced levels of a sphingolipid according to formula (I), more preferably according to formula (II), yet more preferably according to formula (III), most preferably phytosphingosine, sphingosine or sphinganine, or a precursor, a derivative or a pharmaceutically acceptable salt thereof. Such a diet comprises the use of food items or food supplements as described herein and the food items and/or food supplements described herein below may suitably represent nutraceuticals as known in the art.

A sphingolipid according to the formula (I), more preferably according to formula (II), yet more preferably according to formula (III), or a precursor, a derivative or a pharmaceutically acceptable salt thereof, may be used in a food item or food supplement. A food supplement is defined as a composition that can be consumed in addition to the normal food intake and which comprises elements or components that are not or in only minor amounts, present in the normal diet and of which sufficient or increased consumption is desired. The composition of a food item does not necessarily differ much from that of a food supplement.

A food item or food supplement as disclosed herein comprises an amount of sphingolipids according to the formula (I), (II) or (III) that is higher than the amount that would normally or without human intervention occur or be found in said food item or food supplement. This elevated amount of a sphingolipid according to the formula (I), (II) or (III) may arise through specific addition of said sphingolipid to a food composition that does not normally comprise said sphingolipid in said elevated amount, i.e. by enrichment of the food item with said sphingolipid. Alternatively genetic engineering may be used to produce food products comprising said sphingolipid in an elevated amount, for instance by engineering the biosynthetic routes for the production of such sphingolipids in a plant, or yeast or other microorganism used for the production of a food product in such a way that said sphingolipid is produced in said organism in an elevated amount.

Since amounts of sphingolipids such as phytosphingosine, sphingosine, sphingomyelin, lyso-sphingomyelin or sphinganine may differ considerably between various food items there is no general value for the amount which is said to be an elevated amount or of an enriched food item. In general, milk, which normally contains relatively high amounts of sphingomyelin, is said to comprise an elevated amount at higher absolute concentrations than for instance a potato, which contains no or only minute amounts of sphingomyelin.

A sphingolipid-enriched food item or food supplement as described above may suitably comprise 0.01 to 99.9 wt.% of a sphingolipid according to the formula (I), (II) or (III). In a preferred embodiment such a food item or food supplement comprises from 0.05 to 50 wt.%, preferably from 0.05 to 10 wt.%, more preferably from 0.05 tot 5 wt.% of a sphingolipid according to the formula (I), (II) or (III) or derivatives, precursors or acceptable salts thereof.

In order to make a food item or food supplement comprising an elevated amount a sphingolipid according to the formula (I), (II) or (III) suitable for human or animal consumption, the nutritional value, texture, taste or smell may be improved by adding various compounds to said item or supplement. The skilled person is well aware of the different sources of protein, carbohydrate and fat that may be used in food items or food supplements according to the invention and of the possible sweeteners, vitamins, minerals, electrolytes, coloring agents, odorants, flavoring agents, spices, fillers, emulsifiers, stabilizers, preservatives, anti-oxidants, food fibers, and other components for food items that may be added to improve its nutritional value, taste or texture. The choice for such components is a matter of formulation, design and preference. The amount of such components and substances that can be added is known to the skilled person, wherein the choice may e.g. be guided by recommended daily allowance dosages (RDA dosages) for children and adults and animals.

Portions for intake of the food item or food supplement may vary in size and are not limited to the values corresponding to the recommended dosages. The term "food supplement" is herein not intended to be limited to a specific weight or dosage.

A composition of a food item or food supplement as described above may in principle take any form suited for consumption by man or animal. In one embodiment the composition is in the form of a dry powder that can be suspended, dispersed, emulsified or dissolved in an aqueous liquid such as water, coffee, tea, milk, yogurt, stock or fruit juice and alcoholic drinks. To this end, the powder may be provided in unit-dosage form.

In an alternative preferred embodiment a composition in the form of a dry powder is tableted. To that end, a composition for a food supplement according to the invention may very suitably comprise fillers, such as microcrystalline cellulose (MCC) and mannitol; binders such as hydroxypropylcellulose (HPC) and lactose; disintegrants such as starch; and lubricants such as stearic acid; as well as other excipients.

A composition of a food item or food supplement as described above may also be provided in the form of a liquid preparation wherein the solids are suspended, dispersed or emulsified in an aqueous liquid. Such a composition may be admixed directly through a food item or may e.g. be extruded and processed to grains or other shapes.

In an alternative embodiment a food item or food supplement may take the shape of a solid, semi-solid or liquid food item, such as a bar, cookie or a sandwich, spreads, sauces, butter, margarines, dairy products, and the like. Preferably, a sphingolipid according to the present invention is applied in a dairy product, such as for instance a butter or margarine, custard, yogurt, cheese, spread, drink, or pudding or other dessert. The sphingolipid can also be used in butters or fats used for frying and baking, because they are relatively stable and will not be degraded by high temperatures. This characteristic also enables use of the sphingolipid in food items or food supplements which undergo a pasteurization or sterilization treatment. Diet products also constitute preferred embodiments of food items or food supplements according to the invention.

If a food item according to the invention is used as an animal feed, the food item may e.g. be prepared in the form of a powder, a grain, a waffle, a porridge, a block, a pulp, a paste, a flake, a cook, a suspension or a syrup.

For administration to humans the food substance of the invention may very suitably be prepared in the form of a food supplement.

A food item or food supplement for use in a method of to the invention may be prepared by enriching a food item or food supplement with a sphingolipid according to the formula (I) and/or (II) and/or (III), or a precursor, a derivative or a pharmaceutically acceptable salt thereof.

A method for the preparation of a food item or food supplement enriched with a sphingolipid may comprise processing a sphingolipid according to the formula (I), (II) or (III), or a precursor, a derivative or a pharmaceutically acceptable salt thereof in a food item or food supplement, preferably to an amount of 0,01 to 99,9 wt.%, more preferably to an amount of from 0,05 to 50 wt.%, even more preferably to an amount of from 0.05 tot 10 wt.%, and most preferably to an amount of from 0.05 tot 5 wt.%. The amount of sphingolipid processed in a food item suitable for use in a method of the invention depends on the type of sphingolipid and its use and the skilled person is capable of determining this amount in the context of the present disclosure.

The skilled person will understand that the food item may first be prepared separately and then be joined with a sphingolipid to provide a food item wherein said sphingolipid is incorporated in the food item. The food item may be separately prepared by conventional methods such as by mixing, baking, frying, cooking, steaming or poaching and may, if necessary, be cooled prior to joining with the sphingolipid. Alternatively, the sphingolipid may be incorporated as a component in the food item during the preparation thereof.

A food item or food supplement according to the present invention may very suitably be defined as a nutraceutical composition. Nutraceuticals can be defined as natural products that are used to supplement the diet by increasing the total dietary intake of important nutrients. This definition includes nutritional supplements such as vitamins, minerals, herbal extracts, antioxidants, amino acids, and protein supplements. Nutraceutical products fit into the newly created product category of "Dietary Supplements" as established by the F.D.A. in the Dietary Supplement Act of 1994. This act specifically defined dietary supplements to include: vitamins, minerals, herbs or other botanicals, antioxidants, amino acids, or other dietary substances used to supplement the diet by increasing the total dairy intake.

A "nutraceutical composition" is defined herein as a food composition fortified with ingredients capable of producing health benefits. Such a composition in the context of the present invention may also be indicated as foods for special dietary use; medical foods; and dietary supplements. The food item and/or food supplement of the present invention is a nutraceutical composition since it is fortified with one or more sphingolipids according to the invention and since it is capable of preventing and/or treating atherosclerosis, improve blood glucose homeostasis in insulin resistant subjects and/or improve the physiological effects of insulin.

The present invention also relates to a method for preventing and/or treating atherosclerosis in a subject, preferably a human or other mammalian subject, said method comprising administering to said subject a therapeutically and/or prophylactically effective amount of a pharmaceutical composition comprising a sphingolipid according to formula (I), more preferably according to formula (II), yet more preferably according to the formula (III), most preferably phytosphingosine, sphingosine and/or sphinganine or precursors, derivatives or pharmaceutically acceptable salts thereof and a pharmaceutically acceptable carrier, and optionally one or more excipients.

The present invention also relates to the use of a sphingolipid according to formula (I), more preferably according to formula (II), yet more preferably according to the formula (III), most preferably phytosphingosine, sphingosine and/or sphinganine or precursors, derivatives or pharmaceutically acceptable salts thereof for the manufacture of a medicament for deactivating activated immune cells, preferably macrophages and/or T-cells, more preferably T-cells. Also contemplated is the use in food items, supplements and nutraceuticals as part of a diet for eactivating activated immune cells, preferably macrophages and/or T-cells, more preferably T-cells in subjects in need thereof.

An anti-atherosclerosis therapy (i.e. the method for preventing and/or treating atherosclerosis in a subject) may also comprise administering to an otherwise healthy individual, at risk of developing atherosclerosis or its clinical complications, a prophylactically effective amount of the pharmaceutical composition, food item or food supplement described herein. The food item or food supplement may suitably be part of a specific diet for preventing atherosclerosis or its clinical sequellae or for treating subjects with atherosclerosis.

Since atherosclerosis is not a disease condition *per se,* as it will develop gradually in human subjects past the age of 30-50, the risk of acquiring atherosclerosis may be diminished by the administration of a non-prescribed medicament, a food item or a food supplement to an at risk subject by medically non-skilled persons. Many of the food items and food supplements, including nutraceuticals, of the present invention may be sold over-the-counter in health-food shops or chemist's. As such, in one preferred embodiment, the present invention relates to a method of preventing atherosclerosis in a at risk subject as a non-medical method.

In another embodiment, the present invention relates to a method of treating atherosclerosis in a subject as a non-medical method. Such a method of treatment may be performed by the administration of a non-prescribed medicament, a food item or a food supplement to healthy subject in order to slow the progress of atherosclerosis or even to reduce atherosclerosis in persons that do not suffer from a medical condition. As such, in another preferred embodiment, the present invention relates to a method of treating atherosclerosis in a healthy subject as a non-medical method.

Dosages for achieving the anti-atherogenic effects of the pharmaceutical composition, food item or food supplement described herein may easily be determined by the skilled person. For purposes of the present invention, an effective dose will be from about 0.05-5% of the dry food weight in the individual to which it is administered, meaning that for an adult human being the daily dose will be between about 0.01 and 10 grams of sphingolipid.

Preferably a pharmaceutical composition as described above is intended for oral application. Compositions for oral application will usually comprise an inert diluent or an edible carrier. The compositions may be packed in e.g. gelatin capsules or may be tableted in the form of tablets. For oral therapeutic application the active compound may be administered with excipients and e.g. used in the form of powders, sachets, tablets, pills, pastilles or capsules. Pharmaceutically acceptable binders and/or adjuvants may also be comprised as constituents of the pharmaceutical composition.

The powders, sachets, tablets, pills, pastilles, capsules and such may comprise each of the following components or compounds of similar import: a filler such as microcrystalline cellulose (MCC) or mannitol; a binder such as hydroxypropylcellulose (HPC), tragacanth gum or gelatin; an filler such as lactose; a disintegrant such as alginate or starch; a lubricant such as magnesium stearate; a sweetener such as sucrose or saccharose; or a flavoring substance such as peppermint or methyl salicylic acid.

When dosing is in the form of a capsule, the capsule may comprise apart from the elements mentioned above a liquid carrier such as an oil. Dosage form may further be provided with coatings of sugar, shellac or other agents. The components of the pharmaceutical composition are preferably chosen such that they do not reduce the desired working of the sphingolipid.

A sphingolipid according to the formula (I), (II) or (III) or the pharmaceutically acceptable salt thereof may also be administered in the form of e.g. an elixir, a suspension, a syrup, a waffle or a chewing gum.

In a pharmaceutical composition as described above, a sphingolipid according to the formula (I), (II) or (III), or a precursor, a derivative or a pharmaceutically acceptable salt thereof, is used in an amount of from 0.01 to 99.9 % by weight, preferably from 0.01 to 10 wt.%, and more preferably from 0.05 to 5 wt.%.

A pharmaceutical composition according to the invention is intended for reducing the inflammatory response as exemplified, but not limited to the reduction of TNFα release by macrophages and the suppression of the signal transduction pathways in activated T-cells.

The present invention further relates to a method for the preparation of a pharmaceutical composition for preventing and/or treating atherosclerosis, comprising processing or incorporating a sphingolipid according to the formula (I), (II) or (III), or a precursor, a derivative or a pharmaceutically acceptable salt thereof, as an active substance, together with a pharmaceutically acceptable carrier in a pharmaceutical composition.

The preparation of a pharmaceutical composition may very suitably occur by mixing all separate ingredients such as fillers, binders, lubricants and optionally other excipients together with a sphingolipid according to the formula (I), (II) or (III) or a precursor, a derivative or a pharmaceutically acceptable salt thereof, and processing the mixture obtained to a pharmaceutical preparation.

The invention will now be illustrated by way of the following, non-limiting examples.

### EXAMPLE

### Example 1. Effect of sphingolipids on macrophages and T-cells.

### Macrophage culture

Undifferentiated U937 cells were grown at 37 °C at 5% (v/v) CO₂ and 100% relative humidity in medium consisting of RPMI 1640, and supplemented with 10 % Fetal Calf Serum (FCS), 2 mM L-Glutamine, 50 µg/ml Gentamycin and 0.4 % Fungizone. The cells were passaged every three days. Finally the cells were transferred to 96-wells microtiter plates. Differentiation was induced by addition of 10 ng/ml PMA (Phorbol 12-myristate 13-acetate). After 24 hours the medium was changed and refreshed every day and the differentiated macrophages were grown for another 3 days at 37 °C.

### Effect of sphingolipids on LPS-induced TNFα release by macrophage U937 cells

The above described differentiated U937 cells were treated for 30 min with 1 µg/ml or 10 µg/ml sphingolipid (1 mg/mL in ethanol). Then the cells were stimulated with LPS (1 µg/ml) for a period of 4 hours. The TNFα release was measured with the TNF-α cytoset™ from Biosource International, Camarillo, Ca, USA.

### Cell proliferation/viability of macrophage (U937) cells in the presence of sphingolipids

Because sphingolipids can be cytotoxic to cells, the viability of the cells after exposure to the sphingolipids was checked with the MTT (3-(4,5-dimethylthiazolyl-2)-2,5-diphenyltetrazolium bromide) assay. Living cells transform MTT to the purple formazan. During the LPS stimulation of the cells (in 200 µL), in the presence or absence of sphingolipids, 20 µl MTT (1 mg/ml) was added. The medium was removed, the cells were lysed with 100 µL ethanol and the extinction at 590 nm was measured.

### Jurkat (T-cell) culture

Jurkat cells (T-cells) stably transfected with pNFAT-luc (Clontech, BD Bioscience, Becton, Dickinson and Company, Franklin Lakes, NJ USA) in which the hygromycin resistance gene was cloned were used. The cells produce the enzyme luciferase under control of the NFAT (nuclear Factor of Activated T cells) promoter. The NFAT-mediated pathways are stimulated in Jurkat cells by PMA and ionomycin. Cells were grown at 37 °C at 5% (v/v) CO₂ and 100% relative humidity in medium consisting of RPMI 1640, 10 mM HEPES (N-[2-hydroxyethyl]piperazine-N'-[2-ethanesulfonic acid), 10% FCS, 2 mM L-Glutamine and 1 mM Sodium Pyruvate. The medium was changed every 3 days. Finally the cells were transferred to 96-wells plates.

### Cell proliferation/viability of Jurkat cells in the presence of sphingolipids

Because sphingolipids can be cytotoxic to cells, the viability of the cells after exposure to the sphingolipids was checked with the Alamar Blue™ (Biosource) test. Almar blue™ (20 µL) and sphingolipids were added simultaneously. The Fluorescence (excitation at 540 nm and emission at 570 nm) was measured in a Wallac 1420 Victor² multilabel counter (Perkin Elmer Wellesley, MA, USA).

### Effect of sphingolipids on PMA/Ionomycin-induced stimulation of Jurkat cells

A cell suspension of 5 x 10⁵ cells/ml in medium without phenol red was transferred to 96 well microtiter plates (200 µL/well). In triplicate sphingolipids were added (1 or 10 µg/mL). After 30 min PMA (50 ng/ml) and ionomycin (1 µg/ml) was added and the cells were incubated during 6 hours. Then 60 µl cell suspension was mixed with 60µl steady glo reagent (Promega). Luminescence was measured in a Wallac 1450 microbeta liquid scintillation counter.

### Results

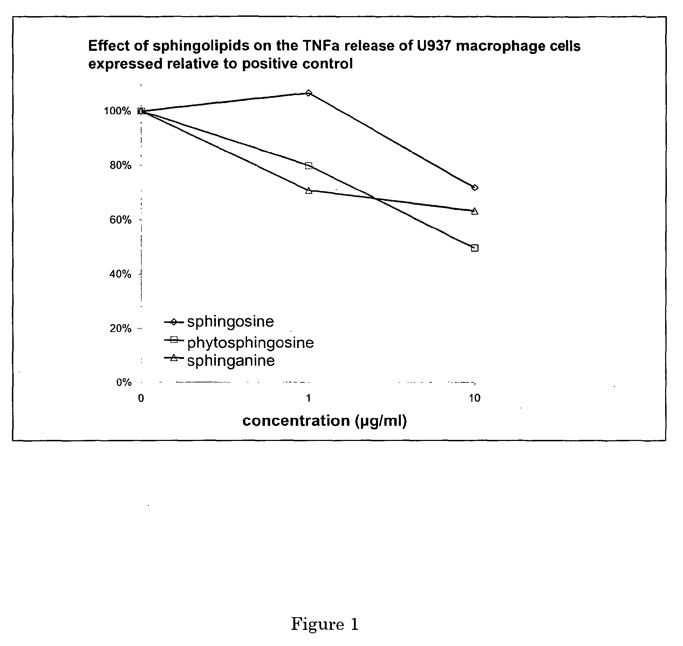

In Figure 1 the reduced release of TNFα by sphingolipids in LPS-stimulated macrophages is shown. The reduction was not due to loss of cell viability by the presence of sphingolipids, since absorption at 590 nm did not decrease in the MTT assay.

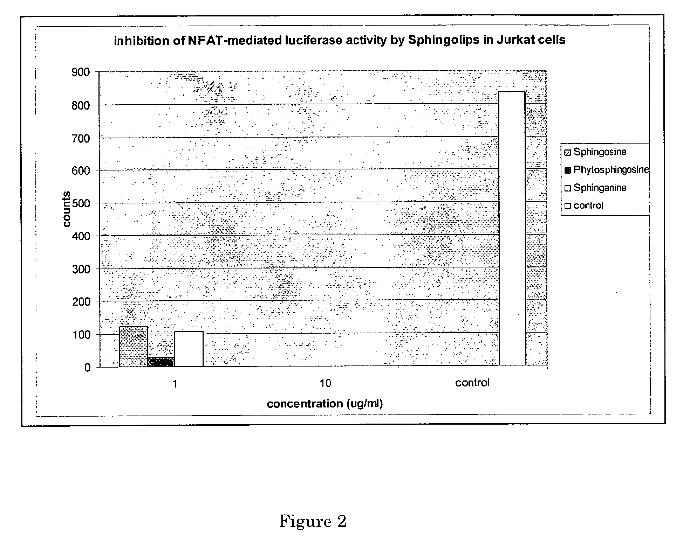

In Figure 2 the influence of sphingosine, sphinganine and phytosphingosine on the NFAT-mediated luciferase activity (i.e. T-cell activation) is shown. It is clear that at 10 µg/mL sphingolipid the PMA/ionomycin-induced activation of the NFAT-mediated signal transduction pathway is completely suppressed. No differences were observed in cell viability as a result of the exposure to sphingolipids.

## Claims

1. Use of a sphingolipid with the general formula (I): 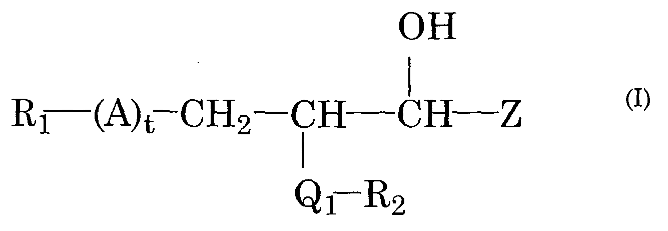
wherein
Z is R₃ or -CH(OH)-R₃;
A is sulphate, sulphonate, phosphate, phosphonate or -C(O)O-;
R₁ is H, hydroxyl, alditol, aldose, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-); and
t is 0 or 1, or a precursor, a derivative or a pharmaceutically acceptable salt thereof, for the manufacture of a medicament for preventing and/or treating atherosclerosis.

2. Use of sphingolipid according to the general formula (II) 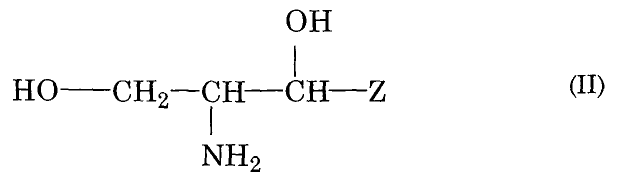
wherein
Z is R₃ or CH(OH)-R₃, and
R₃ is an unsaturated or saturated (C₁-C₃₀) alkyl chain, or a precursor, a derivative or a pharmaceutically acceptable salt thereof,
for the manufacture of a medicament for preventing and/or treating atherosclerosis.

3. Use of sphingolipid according to the general formula (III) 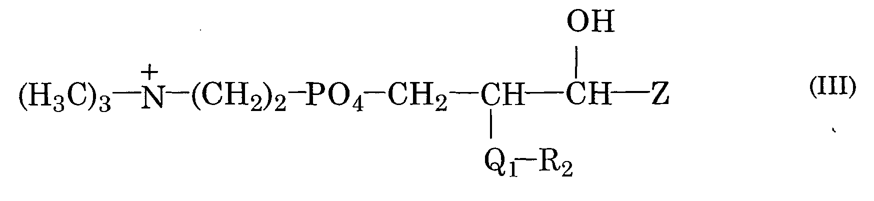
wherein
Z is R₃ or CH(OH)-R₃, preferably R₃;
Q₁ is a primary amine group (-NH₂), a secondary amine group (-NH-) or an amide group (-NH-CO-); preferably an amide group, and
R₂ is H or unsaturated or saturated (C₁-C₃₀) alkyl chain;
R₃ is an unsaturated or saturated (C₁-C₃₀) alkyl chain, preferably an unsaturated (C₁₋C₃₀) alkyl chain,
or a precursor, a derivative or a pharmaceutically acceptable salt thereof,
for the manufacture of a medicament for preventing and/or treating atherosclerosis.

4. Use according to claim 2, wherein said sphingolipid is phytosphingosine, sphingosine and/or sphinganine.

5. Use according to claim 3, wherein said sphingolipid is phytosphingosine.

6. Use of a sphingolipid according to the formula (I) as defined in claim 1 or formula (II) as defined in claim 2, or formula (III) as defined in claim 3, or a precursor or a derivative thereof in food products for preventing and/or treating atherosclerosis and atherosclerosis-related clinical conditions.

7. A method for preventing and/or treating atherosclerosis.in a subject, preferably a human or other mammalian subject, said method comprising administering to said subject a therapeutically and/or prophylactically effective amount of a pharmaceutical composition, said composition comprising a sphingolipid according to the formula (I), more preferably according to formula (II), yet more preferably according to the formula (III), most preferably phytosphingosine, sphingosine or sphinganine, sphingomyelin, lyso-sphingomyelin or precursors, derivatives or pharmaceutically acceptable salts thereof and a pharmaceutically acceptable carrier, and optionally one or more excipients

8. Method for preventing and/or treating atherosclerosis in a healthy subject, said method comprising providing said subject with a diet comprising a food products with enhanced levels of a sphingolipid according to the formula (I), (II) or (III) or a precursor, a derivative or a pharmaceutically acceptable salt thereof.

9. Use of a food product comprising enhanced levels of a sphingolipid according to the formula (I), (II) or (III) or a precursor, a derivative or a pharmaceutically acceptable salt thereof, in a diet for preventing and/or treating atherosclerosis in a healthy subject.

10. Use of a sphingolipid as defined in any one of claims 1-3 for the manufacture of a medicament for deactivating activated immune cells, preferably macrophages and/or T-cells, more preferably T-cells.
